(19) Europäisches Patentamt
European Patent Office
Office européen des brevets

(11) **EP 4 523 519 A1**

(12) **EUROPEAN PATENT APPLICATION**

(43) Date of publication:
**19.03.2025 Bulletin 2025/12**

(21) Application number: **23197453.6**

(22) Date of filing: **14.09.2023**

(51) International Patent Classification (IPC):
**A01G 7/04** $^{(2006.01)}$     **G01N 21/64** $^{(2006.01)}$
**G01N 33/00** $^{(2006.01)}$     **G01N 21/84** $^{(2006.01)}$

(52) Cooperative Patent Classification (CPC):
**A01G 7/045; G01N 21/6486; G01N 33/0098;**
G01N 2021/635; G01N 2021/8466

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**KH MA MD TN**

(71) Applicant: **Gardin Ltd**
**Abingdon, Oxfordshire OX14 4RY (GB)**

(72) Inventor: **GODDING, Julian**
**Bisley, GL6 7AN (GB)**

(74) Representative: **Gill Jennings & Every LLP**
**The Broadgate Tower**
**20 Primrose Street**
**London EC2A 2ES (GB)**

(54) **ENVIRONMENT CONTROL METHOD FOR PLANT GROWTH**

(57)    A method of controlling environmental conditions for plant growth comprises:

making a plurality of sets of chlorophyll fluorescence measurements of a plant at plurality of different measurement times;

calculating a first parameter indicative of a quantum yield of photosynthesis of the plant at each measurement time based on the respective set of chlorophyll fluorescence measurements;

calculating a second parameter indicative of a maximum efficiency of PSII photochemistry of the plant at a plurality of the measurement times based on the respective set of chlorophyll fluorescence measurements;

calculating a third parameter indicative of a photochemical quenching coefficient of the plant at a plurality of the measurement times based on the respective set of chlorophyll fluorescence measurements;

and, in a comparison step (S101, S102, S103, S104, S105, S106, S107, S108, S111, S112, S113, S114, S115, S121, S122, S123, S124, S131, S132, S133, S134, S135, S136, S141, S142, S143, S144, S145, S146, S151, S152, S153, S154, S155, S156, S157, S158, S160, S161, S162), ascertaining a first relationship between the first and second parameters indicative of how the quantum yields of photosynthesis vary with the maximum efficiencies of PSII photochemistry;

ascertaining a second relationship between the first and third parameters indicative of how the quantum yields of photosynthesis vary with the photochemical quenching coefficients;

and either:

generating a control signal indicative of plant growth being limited by gas exchange with the environment when the first and second relationships indicate that the quantum yields of photosynthesis increase more rapidly with, or are more strongly positively correlated to, the maximum efficiencies of PSII photochemistry than the photochemical quenching coefficients;

or generating a control signal indicative of plant growth being limited by light exposure from the environment when the first and second relationships indicate that the quantum yields of photosynthesis increase more rapidly with, or are more strongly positively correlated to, the photochemical quenching coefficients than the maximum efficiencies of PSII photochemistry.

EP 4 523 519 A1

START

S101 — Grow a plant using initial set of environmental conditions.

S102 — Make chlorophyll fluorescence measurements $F_0'$, $F_m'$ and $F'$.

S103 — Have measurements been made with actinic light of ten different intensities?

No → Adjust actinic light intensity. S104

Yes

S105 — Estimate a quantum yield of photosynthesis of the plant for each set of chlorophyll fluorescence measurements.

S106 — Estimate a maximum efficiency of PSII photochemistry of the light-adapted plant for each set of chlorophyll fluorescence measurements.

S107 — Estimate a photochemical quenching coefficient of the plant for each set of chlorophyll fluorescence measurements.

S108 — Perform a first correlation of the estimated quantum yields of photosynthesis with the estimated maximum efficiencies of PSII photochemistry and perform a second correlation of the estimated quantum yields of photosynthesis with the estimated photochemical quenching coefficients.

A

FIG. 1

**Description**

**FIELD OF THE INVENTION**

The invention relates to a method of controlling environmental conditions for plant growth suitable for use in commercial greenhouses, vertical farms and the like.

**BACKGROUND**

In many commercial crop growth contexts, a controllable actinic light source is used to induce photochemistry required for plant growth. Controllable actinic light sources include controllable artificial lights or natural lighting controlled by way of shades or the like. This is normally paired with control of other environmental conditions, such as temperature, pressure, humidity, and $CO_2$ concentration, which all affect gas exchange between the plant and the environment, i.e. uptake of $CO_2$. When growing a crop in these conditions, a grower needs to determine appropriate values to ensure that a crop is getting sufficient light and $CO_2$ for efficient plant growth. In order to efficiently grow a crop, a balance between light and $CO_2$ needs to be found. Generally, assuming sufficient availability of water, plant photosynthesis may be either light limited, i.e. the plant has an abundance of $CO_2$ available to it but not enough light to make full use of that $CO_2$, or may be $CO_2$ limited, in which the plant has an abundance of light but not enough $CO_2$. To increase plant photosynthesis it would be most effective to increase either light or $CO_2$ depending on what is lacking. Even when no additional photosynthesis is desired, operating with either an excess of light or an excess of $CO_2$ is generally inefficient.

It can be difficult for a grower to establish which conditions should be adjusted if they want to promote plant growth, or to establish if plant has an excess of light or $CO_2$. Conventionally, growers will use trial and error to discover a suitable balance of light and $CO_2$ for a particular plant species which typically gives a satisfactory yield and quality. Growers may then adjust environmental conditions for each growth cycle in an ad hoc manner to accelerate or decelerate growth, or increase or decrease growth efficiency, based on judgements of the plant at a particular time.

It is preferable to provide a method of controlling environmental conditions for plant growth that does not require trial and error and which allows a grower to find a suitable balance of light and $CO_2$ resources.

**SUMMARY OF INVENTION**

In accordance with a first aspect of the invention, there is provided a method of controlling environmental conditions for plant growth, the method comprising: making a plurality of sets of chlorophyll fluorescence measurements of a plant at plurality of different measurement times; calculating a first parameter indicative of a quantum yield of photosynthesis of the plant at each measurement time based on the respective set of chlorophyll fluorescence measurements; calculating a second parameter indicative of a maximum efficiency of PSII photochemistry of the plant at a plurality of the measurement times based on the respective set of chlorophyll fluorescence measurements; calculating a third parameter indicative of a photochemical quenching coefficient of the plant at a plurality of the measurement times based on the respective set of chlorophyll fluorescence measurements; and, in a comparison step, ascertaining a first relationship between the first and second parameters indicative of how the quantum yields of photosynthesis vary with the maximum efficiencies of PSII photochemistry; ascertaining a second relationship between the first and third parameters indicative of how the quantum yields of photosynthesis vary with the photochemical quenching coefficients; and either: generating a control signal indicative of plant growth being limited by gas exchange with the environment when the first and second relationships indicate that the quantum yields of photosynthesis increase more rapidly with, or are more strongly positively correlated to, the maximum efficiencies of PSII photochemistry than the photochemical quenching coefficients; or generating a control signal indicative of plant growth being limited by light exposure from the environment when the first and second relationships indicate that the quantum yields of photosynthesis increase more rapidly with, or are more strongly positively correlated to, the photochemical quenching coefficients than the maximum efficiencies of PSII photochemistry.

The present method is based on the following observations. Quantum yield of photosynthesis, i.e. the ratio of the number of photons emitted to the number of photons absorbed, is affected by both light intensity and $CO_2$ availability, since it is affected by both photochemical and non-photochemical quenching processes. Photochemical quenching is the process by which absorbed photons are converted into chemical energy used to carry out photosynthesis. Non-photochemical quenching is the process by which absorbed photons are converted into heat. As photochemical quenching increases, quantum yield increases. As non-photochemical quenching increases, quantum yield decreases. Non-photochemical quenching is inversely proportional to maximum efficiency of PSII photochemistry and so as maximum efficiency of PSII photochemistry increases, so does quantum yield. Chlorophyll fluorescence measurements allow for estimation of quantum yield, photochemical quenching, and maximum efficiency of PSII photochemistry. Thus, the present methods uses chlorophyll fluorescence measurements in order to observe how quantum yield increases with maximum efficiency of PSII photochemistry (light adapted) and with photochemical quenching. If it is observed that an increase in maximum efficiency of PSII photochemistry produces a stronger increase in quantum yield than a corresponding increase in photochemical quenching, this may be taken to indicate that the plant already has an excess of light and is thus $CO_2$ limited. On the other hand, if it is observed that an increase in photochemical quenching produces a stronger increase in quantum yield than a corresponding increase in maximum efficiency of PSII photochemistry, this may be taken to indicate that the plant has an excess of $CO_2$ and is thus light limited, i.e. has the capacity to take on more light for useful photochemistry.

In the present method, parameters indicative of quantum yield, maximum efficiency of PSII photochemistry and photochemical quenching coefficient are calculated from the measurements for each of the relevant measurement times. The result will be that a plurality of first parameters that can be compared with a corresponding plurality of second parameters and compared with a corresponding plurality of third parameters. It will be appreciated that one way such parameters may be calculated is by estimating a quantum yield of photosynthesis of the plant at each measurement time based on the respective set of chlorophyll fluorescence measurements, estimating a maximum efficiency of PSII photochemistry of the plant at a plurality of the measurement times based on the respective set of chlorophyll fluorescence measurements, and estimating a photochemical quenching coefficient of the plant at a plurality of the measurement times based on the respective set of chlorophyll fluorescence measurements. Ways of estimating these values will be discussed below. However, it should also be noted that the present method does not require directly estimating these values. For example, as a way of calculating the second parameter, the method could involve estimating a non-photochemical quenching coefficient of the plant at a plurality of the measurement times based on the respective set of chlorophyll fluorescence measurements. This is because, as stated above, non-photochemical quenching is inversely proportional to maximum efficiency of PSII photochemistry. All that is required is that the first, second and third parameters indicative of quantum yield, maximum efficiency of PSII photochemistry and photochemical quenching coefficient allow it to be ascertained how the quantum yields of photosynthesis vary with the maximum efficiencies of PSII photochemistry and how the quantum yields of photosynthesis vary with the photochemical quenching coefficients. In the case where the parameters are calculated by estimating the values, the way the values vary can be immediately ascertained from the values estimated at each measurement time. On the other hand, in the example where a non-photochemical quenching coefficient is estimated, ascertaining how the quantum yields of photosynthesis vary with the non-photochemical quenching coefficient indirectly ascertains how the quantum yields of photosynthesis vary with the maximum efficiencies of PSII photochemistry due to the inverse relationship between non-photochemical quenching and maximum efficiency of PSII photochemistry.

In this method, a plurality of sets of chlorophyll fluorescence measurements of a plant are made at plurality of different measurement times. The sets of chlorophyll fluorescence measurements are preferably made by a chlorophyll fluorescence measurement device. By making sets of measurements at different times, this allows for photochemical and maximum efficiency of PSII photochemistry to passively change or to be actively changed as part of the method, so that it can be observed how the changes in photochemical quenching and maximum efficiency of PSII photochemistry affect quantum yield. Each set of measurements should include chlorophyll fluorescence measurements sufficient to enable calculation of a respective first parameter indicative of quantum yield, since quantum yield needs to be tracked against both photochemical quenching and maximum efficiency of PSII photochemistry. In principle, some sets of measurements could only include chlorophyll fluorescence measurements sufficient to calculate respective second parameters for a maximum efficiency of PSII photochemistry or respective third parameters for a photochemical quenching coefficient, but not both; however, in practice, the method will generally comprise calculating a second parameter indicative of a maximum efficiency of PSII photochemistry of the plant at each measurement time and calculating a third parameter indicative of a photochemical quenching coefficient of the plant at each measurement time. In other words, preferably, from each set of chlorophyll fluorescence measurements, it will be possible to calculate a first parameter indicative of a quantum yield of photosynthesis of the plant, a second parameter indicative of maximum efficiency of PSII photochemistry and a third parameter indicative of the photochemical quenching coefficient.

The method then involves making a series of calculations based on the measurements and performing a comparison. These steps are preferably performed by a controller, such as a computer system that receives the measurement values.

A control signal indicative of plant growth being limited by gas exchange with the environment is generated when the first and second relationships indicate that the quantum yields of photosynthesis increase more rapidly with, or are more strongly positively correlated to, the maximum efficiencies of PSII photochemistry than the photochemical quenching coefficients. A control signal indicative of plant growth being limited by light exposure is generated when the opposite relationship is observed. It is noted that these control signals may be outputted either based on the rate of the increase in quantum yield or based on the strength of a positive correlation. For example, if it is assumed that there is a linear correlation between quantum yield and each of photochemical quenching and maximum efficiency of PSII photochemistry, then the assessment may be based on either the slope of the correlation or the strength of the correlation. The slope of the correlations may provide an indication of which of photochemical quenching and maximum efficiency of PSII photochemistry is associated with a more rapid increase in quantum yield. However, in some circumstances, it may be preferred to select the stronger positive correlation, i.e. in which the data is a closer fit to the correlation, since this may more reliably achieve the desired effect on quantum yield, i.e. increasing or decreasing by a desired amount. The control signals are preferably generated by the controller on the basis of the comparison step. It will be appreciated that the controller should be able to selectively output a control signal indicative of plant growth being limited by gas exchange with the environment or a control signal indicative of plant growth being limited by light exposure from the environment based on whether quantum yield is observed to increase more rapidly with, or be more strongly positively correlated to, the photochemical quenching coefficient or maximum efficiency of PSII photochemistry.

Generally, a plant to which the present method is to be applied will be grown with set environmental conditions prior to the taking of the chlorophyll fluorescence measurements. For example, the method may comprise a first step of growing a plant using an initial set of environmental conditions, which may include a set actinic light intensity, a set temperature, a set pressure, and a set $CO_2$ concentration. The term "set" here does not imply that the grower is able to manually set each value, but typically, one or more of these set environmental conditions will be adjustable by the grower, and the purpose of the method will be to determine which of these environmental conditions should be adjusted relative to the initial set values. Other environmental conditions may not be adjustable, e.g. the set pressure may be atmospheric pressure with no option for the grower to adjust, depending on the growing set up. The present method is applicable to plants grown in natural light, i.e. sunlight, artificial light, or supplemental lighting, which uses artificial light to supplement natural light.

Any known technique for making chlorophyll fluorescence measurements may be used. However, preferably, making each set of chlorophyll fluorescence measurements comprises using a measurement light source to emit substantially collimated or convergently focussed light to irradiate a measurement area of the plant with measurement light, and using an optical sensor to detect chlorophyll fluorescence from the measurement area. The measurement light source is preferably a laser, preferably an adjustable power laser, wherein preferably the laser is configured to irradiate a spot-shaped area with light. The term "substantially collimated" will be understood to encompass a light source with a beam divergence of 10° or less. More tightly collimated light will, of course, be preferred, and indeed, it will be preferred to have a beam divergence of 5° or less, more preferably 2° or less, even more preferably 1° or less, most preferably 0.1 ° or less. Commercially available lasers will typically have a beam divergence of much less than 0.1°. The use of substantially collimated or convergently focussed light allows measurements to be using a light source positioned at a distance, preferably of at least 0.1 m, more preferably from a distance of at least 0.2 m, even more preferably from a distance of at least 0.5 m, e.g. fixed above a plant. Preferably, the optical sensor is also positioned at a distance, preferably of at least 0.1 m, more preferably from a distance of at least 0.2 m, even more preferably from a distance of at least 0.5 m, e.g. fixed above a plant. This is compared with many conventional chlorophyll fluorescence measurement techniques, which involve devices that are manually clipped on to leaves in order to take a measurement, or which bathe a crop in measurement light.

The optical sensor preferably comprises an imaging optical sensor, such as a camera, configured to capture an image of the measurement area. This is preferred for a number of reasons. Firstly, this allows the position of the measurement area within the field of view of the sensor to be determined, which may allow for distance to the measurement area to be determined. Additionally, the images captured during the chlorophyll fluorescence measurements can be used to construct an image of the crop canopy and asses the health of the plants, which can be useful for other aspects of plant growth. While an imaging sensor is preferred, chlorophyll fluorescence could also be measured with a non-imaging system sensor. Indeed, a non-imaging sensor may be preferred in some contexts. For example, a non-imaging sensor may be able to achieve higher measurement frequencies, e.g. if it outputs an analogue signal. Suitable optical sensors may include silicon photodetectors and/or a photodiode.

Whether an imaging or non-imaging optical sensor is used, it is preferable that an optical sensor with a small field of view is used. In particular, the optical sensor may receive light from a field of view of no more than 400 $deg^2$, preferably no more than 200 $deg^2$, more preferably no more than 50 $deg^2$, most preferably no more than 25 $deg^2$. A typical camera that may be used as the optical sensor may have a field of view of 18° x 12° or less, or more preferably 6° x 4°.

While the chlorophyll fluorescence measurements could be made using a fixed measurement light source and optical sensor, i.e. configured to irradiate the same area of the plant on each occasion, it is preferable that the method can be performed on different areas of a plant or even on different plants within a crop. This would allow the method to compensate for plant growth or even repeat the method for different plants in a crop to base the method on the response of multiple plants. Therefore, preferably, the method comprises identifying a target measurement area and using an actuator to controllably direct the light from the measurement light source to irradiate the target measurement area, and preferably making each set of chlorophyll fluorescence measurements at the target area. The movement may typically involve an actuator that is configured to move the light source so as to direct the light from the light source. That is, by moving the whole measurement light source, it is possible to change the area irradiated with light from that light source. Alternatively, or additionally, the actuator may be configured to move one or more optical elements, such as mirrors, relative to the measurement light source so as to direct the light from the measurement light source. The use of, for example, a scanning mirror or a light-steering microelectromechanical system may allow the light from the measurement light source to be directed between different areas without movement of the light source.

While a steerable measurement light source could be used with a fixed optical sensor, e.g. with a wide field of view, preferably, the method comprises an actuator, preferably the same actuator used to controllably direct the light from the measurement light source, to move the optical sensor and/or to move one or more optical elements, such as mirrors, relative to the optical sensor so as to enable the optical sensor to detect chlorophyll fluorescence from the target measurement area. This enables for smaller field of view sensors to be used while still allowing measurements to be made over a wide area. The use of a single actuator to direct both the measurement light source and the optical sensor means that the hardware used to perform the method can be smaller and less expensive to produce.

The present method generates the control signals in a comparison step. In accordance with a preferred embodiment, ascertaining the first relationship comprises performing a first correlation, which comprises correlating the first parameter (e.g. the estimated quantum yields of photosynthesis) with the second parameter (e.g. the estimated maximum efficiencies of PSII photochemistry), and ascertaining the second relationship comprises performing a second correlation, which comprises correlating the first parameter (e.g. the estimated quantum yields of photosynthesis) with the third parameter (e.g. the estimated photochemical quenching coefficients), and wherein the comparison step comprises comparing the first and second correlations. The first and second correlations may preferably be Pearson correlations. The first and second correlations may also be correlations of logarithms, for example logarithms of the estimated quantum yields of photosynthesis, logarithms of the estimated maximum efficiencies of PSII photochemistry, and logarithms of the estimated photochemical quenching coefficients, preferably base ten logarithms. This may ensure that the first and second correlations are linear correlations. The comparison step may comprise comparing a correlation coefficient from the first correlation with a correlation coefficient from the second correlation, and may comprise either: generating the control signal indicative of plant growth being limited by gas exchange with the environment when the first correlation coefficient is greater than the second correlation coefficient; or generating the control signal indicative of plant growth being limited by light exposure from the environment when the second correlation coefficient is greater than the first correlation coefficient. As indicated above, this may make the selection based on the data that is a closer fit to a straight line. The present handling of the comparison, i.e. comparing correlations, simplifies the data processing that must be done and so makes the process quick to run.

As indicated above, in order to determine which of the maximum efficiency of PSII photochemistry and the photochemical quenching coefficient produces a more rapid or more strongly correlated increase in quantum yield, it is necessary to induce some change in the maximum efficiency of PSII photochemistry and photosynthetic quenching coefficient. While this could be done simply by allowing time for the values to naturally change, preferably, the method further comprises irradiating a plant with actinic light using an adjustable light source before and/or while making at least some of the plurality of the sets of chlorophyll fluorescence measurements, wherein the intensity of actinic light for at least two of those sets of chlorophyll fluorescence measurements is different, wherein preferably the intensity of actinic light for each of those sets of chlorophyll fluorescence measurements is different. The intensity of actinic light for the first set of measurements will preferably be the initial set actinic light intensity, i.e. the intensity of light that is being used to grow the plant prior to commencing the chlorophyll fluorescence measurements. Adjusting the intensity of actinic light is a convenient way to induce changes in quantum yield, maximum efficiency of PSII photochemistry and the photochemical quenching coefficient in order to enable sufficient data for the comparison to be performed. It should be noted that, in the present context, an adjustable actinic light source may include a natural light source, i.e. the sun, paired with adjustable shutters or shades, an adjustable artificial light source, or a supplemental light system, in which an adjustable artificial light source is used to supplement natural light. In the latter, adjusting light intensity comprises adjusting the artificial light source based on any variation in the natural light source to achieve a desired light intensity, with continuous adjustments being required even when attempting to maintain an unchanging light intensity.

In accordance with the present method, each set of chlorophyll fluorescence measurements is made at a different measurement time. This is to allow for changes in quantum yield and the quenching coefficients to occur. Preferably each set of chlorophyll fluorescence measurements is at least 30 seconds apart, preferably at least one minute apart. This allows time for the plant to adjust to changes in light and/or for changes to passively occur.

Preferably each set of chlorophyll fluorescence measurements is made on the same target measurement area of the plant. As mentioned above, the plurality of sets of chlorophyll fluorescence measurements may subsequently be repeated at one or more different target measurement areas of the plant, so that the comparison may be based on more data.

The control signals that are generated may be used for a number of different purposes. In general, the control signals may be used to either automatically adjust the environmental conditions for plant growth, or may be used to inform a grower and allow manual adjustment of the environmental conditions. A significant advantage of the present method in the context of a manual adjustment is that the method may be repeated after each manual adjustment to enable a grower to arrive at a well-balanced set of environmental conditions that also achieve an effect that may be desired by the grower, e.g. increasing yield, maintaining yield while increasing efficiency of resource use, or even slowing growth to arrive at a target yield at a target date.

One embodiment further comprises irradiating a plant with actinic light having a set intensity using an adjustable light source before making the plurality of sets of chlorophyll fluorescence measurements, and generating a control signal indicative of plant growth being limited by gas exchange with the environment comprises generating a control signal to decrease the set intensity of actinic light, and/or generating a control signal indicative of plant growth being limited by light exposure from the environment comprises generating a control signal to increase the set intensity of actinic light. In this embodiment, the method may be used to automatically adjust light source to make efficient use of the current level of gas exchange. This therefore involves increasing the intensity of light if the plant has an excess of $CO_2$ or decreasing the intensity of light if the plant has an excess of light.

In another embodiment, generating a control signal indicative of plant growth being limited by gas exchange with the environment comprises generating a control signal to adjust one or more environmental conditions to increase gas exchange with the environment, and/or generating a control signal indicative of plant growth being limited by light exposure from the environment comprises generating a control signal to adjust one or more environmental conditions to decrease gas exchange with the environment. As mentioned above, the one or more environmental conditions may include one or more of temperature, humidity, pressure, and $CO_2$ concentration of the environment. In particular, these are the environmental conditions that may change gas exchange. Generally increasing or decreasing temperature, humidity, pressure, and $CO_2$ concentration will produce a respective increase or decrease in gas exchange. Light intensity is also considered an environmental condition that may be adjusted in the present method; however, light intensity will have a less immediate effect on gas exchange, and so this condition should not be adjusted where the aim is to increase or decrease gas exchange.

It will be appreciated that the above examples may be combined where the method is being performed in an environment in which it is possible to adjust both lighting and gas exchange parameters. For example, if the method is being undertaken with an objective to increase plant growth then, based on the comparison, generating a control signal indicative of plant growth being limited by gas exchange with the environment may comprise generating a control signal to adjust one or more environmental parameters to increase gas exchange with the environment, and/or generating a control signal indicative of plant growth being limited by light exposure from the environment may comprise generating a control signal to increase the set intensity of actinic light. Likewise, with an objective of increasing efficiency or decreasing plant growth, generating a control signal indicative of plant growth being limited by gas exchange with the environment may comprise generating a control signal to decrease the set intensity of actinic light, and/or generating a control signal indicative of plant growth being limited by light exposure from the environment may comprise generating a control signal to adjust one or more environmental parameters to decrease gas exchange with the environment.

The above embodiments automatically adjust environmental conditions based on the comparison. However, as mentioned, the method may also be used to inform manual adjustments. Accordingly, generating a control signal indicative of plant growth being limited by gas exchange with the environment may comprise generating a control signal that causes output of a recommendation to decrease an actinic light intensity and/or to adjust one or more environmental parameters so as to increase gas exchange with the environment, and/or generating a control signal indicative of plant growth being limited by light exposure from the environment may comprise generating a control signal that causes output of a recommendation to increase an actinic light intensity and/or to adjust one or more environmental parameters so as to decrease gas exchange with the environment. Means for outputting a recommendation will be discussed below.

In embodiments that comprise automatically adjusting environmental parameters so as to increase or decrease gas exchange, or outputting a recommendation to do so, the method may further comprise taking one or more measurements of the environment, said one or more measurements including one or more of taking a temperature measurement of the environment using a temperature sensor, taking a humidity measurement of the environment using a humidity sensor, taking a pressure measurement of the environment using a pressure sensor, and taking a $CO_2$ concentration measurement of the environment using a $CO_2$ concentration sensor, and generating a control signal to adjust one or more environmental parameters or generating a control signal that causes output of a recommendation to adjust one or more environmental parameters is further based on the one or more measurements of the environment. In accordance with this method, an adjustment or recommendation may be made with knowledge of the current conditions. For example, any adjustable one or more of temperature, humidity, pressure and $CO_2$ concentration may have

predefined operational ranges, and the adjustment or recommendation may be made based on a comparison of the measurement with the operational range. For example, if it is observed that temperature is relatively low in its operational range, then a recommendation to increase temperature may be output if it is desired to increase plant growth and the plant is determined to be $CO_2$ limited. The cost of increasing any of these adjustable parameters may also be factored in.

In embodiments that comprise automatically adjusting actinic light intensity or recommending such adjustments, the method may further comprise taking a measurement of the intensity of actinic light, and generating a control signal to increase or decrease the set intensity of actinic light or generating a control signal that causes output of a recommendation to increase or decrease an actinic light intensity is further based on the measurement of the intensity of actinic light. While a measurement could be taken with a dedicated light sensor, e.g. a photosynthetic photon flux density (PPFD) sensor, preferably the measurement is made using the same optical sensor used to make the chlorophyll fluorescence measurements. This may comprise capturing one or more images of the plant using the optical sensor, and determining the intensity of light based on the brightness of at least part of the (or each) image. For example, the intensity of light may simply be determined by analysing the pixel values of the pixel in the captured images. As indicated above, this assessment may be based on one image, or may need to be based on several images, e.g. if pulse width modulated grow lights are for the light source. Alternatively, the exposure time may be adjusted to accommodate for pulse width modulated grow lights. The measurement of light intensity may be an absolute measurement, or simply a relative measurement, e.g. compared to other intensities with which the light may operate. As an alternative to measuring the light intensity, in some embodiments, a controller performing the method may be operationally linked to the light source and may obtain an absolute or relative measure of light intensity this way.

As indicated above, the method may comprise outputting a recommendation to a grower. Generally, this will require some signal being sent to a grower. Therefore, preferably, generating a control signal indicative of plant growth being limited by gas exchange with the environment may comprise generating a control signal that causes output of a visual and/or audible indication that plant growth is limited by gas exchange with the environment, and/or wherein generating a control signal indicative of plant growth being limited by light exposure from the environment comprises generating a control signal that causes output of a visual and/or audible indication that plant growth is limited by light exposure from the environment, wherein preferably the or each visual indication comprises displaying information on a display screen. These indications will be understood to include an indication of the state of the crop, i.e. light or

$CO_2$ limited, or an indication of an appropriate intervention in light of that state, i.e. an indication that lighting should be increased or gas exchange decreased if the crop is light limited of that gas exchange should be increased or lighting decreased if the crop is $CO_2$ limited. The display screen may be a display screen of a local computer system that performs the method, or could be a remote display screen or a personal device of the grower, such as their mobile phone.

In some embodiments that perform the comparison using correlation coefficients, visual indication that is output may comprise displaying of a value derived by substituting one correlation coefficient from the other. For example, a value may be displayed that is the correlation coefficient from the first correlation minus the correlation coefficient from the second correlation. In such a case, output of a number less than 0 may indicate that the plant is light limited, whereas output of a number greater than 0 may indicate that the plant is $CO_2$ limited. Alternatively, the two correlation coefficients may be displayed separately on the screen, or a graphical representation of the correlations may be displayed.

In most embodiments, at least two of the sets of chlorophyll fluorescence measurements include a measurement of a maximal fluorescence of the plant in a light-adapted state, $F_m'$, and a baseline fluorescence of the plant in a light-adapted state, $F'$, and preferably a minimal fluorescence of the plant in a light-adapted state, $F_0'$. Preferably, each set of chlorophyll fluorescence measurements includes a measurement of the maximal fluorescence of the plant in a light-adapted state, $F_m'$, and the baseline fluorescence of the plant in a light-adapted state, $F'$, and preferably a measurement of a minimal fluorescence of the plant in a light-adapted state, $F_0'$. A measurement of minimal fluorescence of a plant in a light-adapted state is generally obtained by temporarily switching off actinic light for a plant that is light-adapted and observing the decay in fluorescence to the minimal value, $F_0'$, when all PSII reaction centres are in an open state. This measurement is not always possible to directly perform, e.g. actinic light cannot be switched off, such as when the sun is some or part of the actinic light source. A measurement of the maximal fluorescence of a plant in this light-adapted state, $F_m'$, is measured by exposing the light-adapted plant to a high intensity pulse, which closes all PSII reaction centres. Finally, after the fluorescence spikes, it will be quenched by photochemical and non-photochemical processes as it adapts to the light and emits a baseline fluorescence, $F'$.

With the above measurements, one way of performing the calculations of the parameters required in the present method is to calculate the first parameter by estimating the quantum yield of photosynthesis for each measurement time by calculating $\dfrac{F_m' - F'}{F_m'}$, to calculate the second parameter by estimating the maximum efficiency of PSII photochemistry for each measurement time by calculating $\frac{F_m' - F_0'}{F_m'}$, and/or to calculate the third parameter by estimating the photochemical quenching coefficient for each measurement time by calculating $\frac{F_m' - F'}{F_m' - F_0'}$. This requires either making a measurement of the minimal fluorescence of the plant in a light-adapted state, $F_0'$, in at least two of the sets of chlorophyll fluorescence measurements, or estimating the minimal fluorescence of the plant in a light-adapted state, $F_0'$, based on other chlorophyll fluorescence measurements of at least two of the sets of chlorophyll fluorescence measurements. One way of estimating $F_0'$ is by using the Oxborough and Baker equation, which allows $F_0'$ to be estimated from other measurement values more readily obtainable:

$$F_0' = F_0 / \left[ \frac{F_v}{F_m} + \frac{F_0}{F_m'} \right]$$

where $F_0$ is the minimal fluorescence of the plant in a dark-adapted state, $F_m$ is the maximal fluorescence of the plant in a dark-adapted state, and $F_v$ is given by $F_m - F_0$. The dark-adapted measurements may be taken from a measurement of the same plant made the previous night, when the plant was dark adapted. While the above calculations are preferred, it will be appreciated that any means of estimating these values would be possible. As mentioned above, an example of an alternative second parameter is a non-photochemical coefficient, which may be estimated by calculating $\frac{F_m - F_m'}{F_m'}$.

In accordance with a second aspect of the invention, there is provided a system for controlling environmental conditions for plant growth, the system comprising: a chlorophyll fluorescence measurement device configured to make a plurality of sets of chlorophyll fluorescence measurements of a plant at plurality of different measurement times; and a controller, the controller configured to: calculate a first parameter indicative of a quantum yield of photosynthesis of the plant at each measurement time based on the respective set of chlorophyll fluorescence measurements; calculate a second parameter indicative of a maximum efficiency of PSII photochemistry of the plant at a plurality of the measurement times based on the respective set of chlorophyll fluorescence measurements; calculate a third parameter indicative of a photochemical quenching coefficient of the plant at a plurality of the measurement times based on the respective set of chlorophyll fluorescence measurements; and to perform a comparison comprising ascertaining a first relationship between the first and second parameters indicative of how the quantum yields of photosynthesis vary with the maximum efficiencies of PSII photochemistry, ascertaining a second relationship between the first and third parameters indicative of how the quantum yields of photosynthesis vary with the photochemical quenching coefficients, and either: generate a control signal indicative of plant growth being limited by gas exchange with the environment when the first and second relationships indicate that the quantum yields of photosynthesis increase more rapidly with, or are more strongly positively correlated to, the maximum efficiency of PSII photochemistry than the photochemical quenching coefficients; or generate a control signal indicative of plant growth being limited by light exposure from the environment when the first and second relationships indicate that the quantum yields of photosynthesis increase more rapidly with, or are more strongly positively correlated to, the photochemical quenching coefficients than the maximum efficiency of PSII photochemistry.

The system according to the second aspect of the invention corresponds to a system for performing the method according to the first aspect of the invention. Accordingly, each of the preferred features described above is equally applicable to the system according to the present aspect.

In accordance with a third aspect of the invention, a method of controlling environmental conditions for plant growth comprises: making a plurality of sets of chlorophyll fluorescence measurements of a plant at plurality of different measurement times; calculating a first parameter indicative of a quantum yield of photosynthesis of the plant at each measurement time based on the respective set of chlorophyll fluorescence measurements; calculating a second parameter indicative of a maximum efficiency of PSII photochemistry of the plant at a plurality of the measurement times based on the respective set of chlorophyll fluorescence measurements; calculating a third parameter indicative of a photochemical quenching coefficient of the plant at a plurality of the measurement times based on the respective set of chlorophyll fluorescence measurements; ascertaining a first relationship between the first and second parameters indicative of how the quantum yields of photosynthesis vary with the maximum efficiencies of PSII photochemistry; ascertaining a second relationship between the first and third parameters indicative of how the quantum yields of photosynthesis vary with the photochemical quenching coefficients; and displaying on a display screen the ascertained first relationship and displaying on the display screen the ascertained second relationship.

This method corresponds to the method according to the first aspect of the invention, except in that instead of performing a comparison step, the system displays the ascertained relationships in such a way that the relationship between the estimated quantum yields of photosynthesis, the maximum efficiency of PSII photochemistry and the photochemical quenching coefficient can be observed by a viewer.

The step of displaying on the display screen the first and second relationships may comprise performing a first correlation, which comprises correlating the first parameter with the second parameter, and performing a second correlation, which comprises correlating the first parameter with the third parameter, and displaying the results of the first and second correlations on the display screen, wherein preferably the first and second correlations are Pearson correlations, preferably of logarithms of the calculated parameters.

According to a fourth aspect of the invention, there is provided a system for assessing environmental conditions for plant growth, the system comprising: a chlorophyll fluorescence measurement device configured to make a plurality of sets of chlorophyll fluorescence measurements of a plant at plurality of different measurement times; a controller, the controller configured to: calculate a first parameter indicative of a quantum yield of photosynthesis of the plant at each measurement time based on the respective set of chlorophyll fluorescence measurements; calculate a second parameter indicative of a maximum efficiency of PSII photochemistry of the plant at a plurality of the measurement times based on the respective set of chlorophyll fluorescence measurements; calculate a third parameter indicative of a photochemical quenching coefficient of the plant at a plurality of the measurement times based on the respective set of chlorophyll fluorescence measurements, ascertain a first relationship between the first and second parameters indicative of how the quantum yields of photosynthesis vary with the maximum efficiencies of PSII photochemistry, and ascertain a second relationship between the first and third parameters indicative of how the quantum yields of photosynthesis vary with the photochemical quenching coefficients; and a display screen, wherein the controller is further configured to cause the display screen to display the ascertained first and to display the ascertained second relationship.

This system corresponds to a system for performing the method according to the third aspect of the invention.

## BRIEF DESCRIPTION OF DRAWINGS

The invention will now be described with reference to the accompanying Figures, of which:

Figure 1 is a flow diagram illustrating a first part of a method according to the invention;
Figure 2 is a flow diagram illustrating a second part of a method according to the invention;
Figure 3 is a flow diagram illustrating a variant of a second part of a method according to the invention;
Figure 4 is a flow diagram illustrating another variant of a second part of a method according to the invention;
Figure 5 is a flow diagram illustrating another variant of a second part of a method according to the invention;

Figures 6 to 8 are flow diagrams illustrating another variant of a second part of a method according to the invention;
Figure 9 is a schematic illustration of a system suitable for performing the method of Figures 1 to 8;
Figure 10 is a perspective view of a system suitable for performing the method of Figures 1 to 8;
Figure 11 is a perspective view of part of the system of Figure 9; and
Figure 12 is a side view of part of the system of Figure 9.

## DETAILED DESCRIPTION

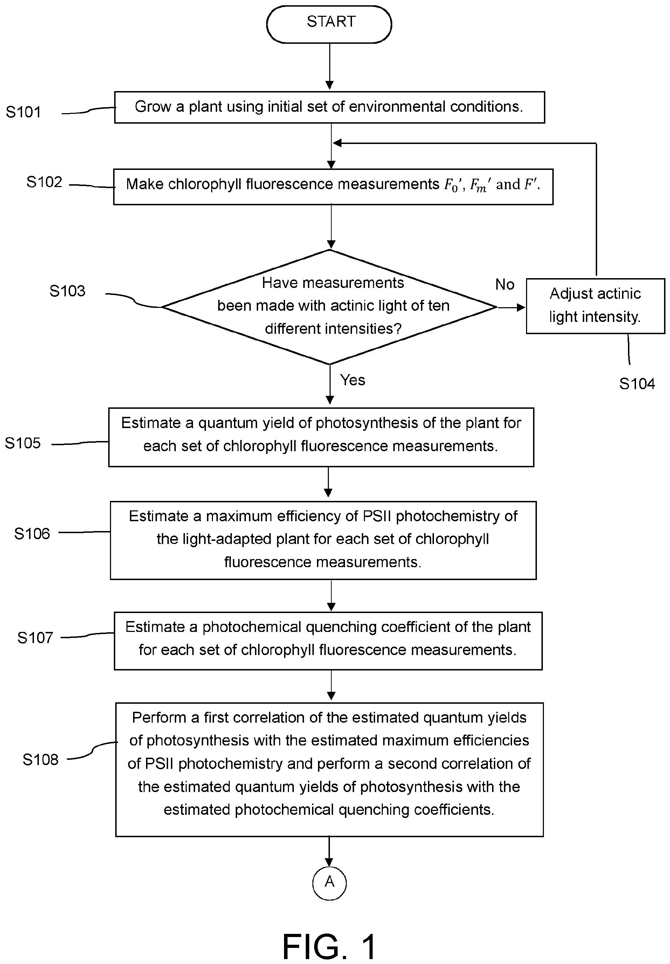

A first part of a method according to the invention will now be described with reference to Figure 1.

In step S101, a plant or crop of plants is grown using an initial set of environmental conditions. These environmental conditions may include an intensity of actinic lighting that may be set by a grower on an actinic lighting system. The actinic lighting system will typically be an LED lighting system producing light in the wavelength range 400-700 nm, but could also include light outside of this range. The lighting system will typically be adjustable to adjust the intensity of light, i.e. the photosynthetic photon flux density (PPFD). The environmental conditions also include environmental conditions that affect gas exchange between the plant and the surrounding air, which include the air temperature, air humidity, air pressure, and $CO_2$ concentration of the air in the environment. One or more of these environmental conditions affecting gas exchange will typically be adjustable by the grower. The grower may grow the crop with these environmental conditions set as desired by the grower based on their priorities for the crop. Some or all of the environmental conditions may also be, in part, influenced by outside factors, such as the weather.

Step S102 represents the beginning of the method for controlling the environmental conditions of the crop. In this step, a plurality of chlorophyll fluorescence measurements are made. In particular, the measurements made here are a measurement of a maximal fluorescence of the plant in a light-adapted state, $F_m'$, a baseline fluorescence of the plant in a light-adapted state, $F'$, and a minimal fluorescence of the plant in a light-adapted state, $F_0'$. A description of the theory behind chlorophyll fluorescence analysis can be found in "Chlorophyll fluorescence analysis: a guide to good practice and understanding some new applications.", by E. Murchie and T. Lawson, Journal of experimental botany, 64 13 (2013): 3983-98, DOI:10.1093/jxb/ert208. Making a measurement of a minimal fluorescence of the plant in a light-adapted state, $F_0'$, assumes that it is possible to turn off the actinic light source. This is possible in many indoor farms and vertical greenhouses. Alternatively, $F_0'$ can be estimated using the Oxborough and Baker equation, given above, using a combination of light-adapted measurements taken now and dark-adapted measurements that were taken of the same plant the night before and stored in a memory of the measurement system (which will be described further below).

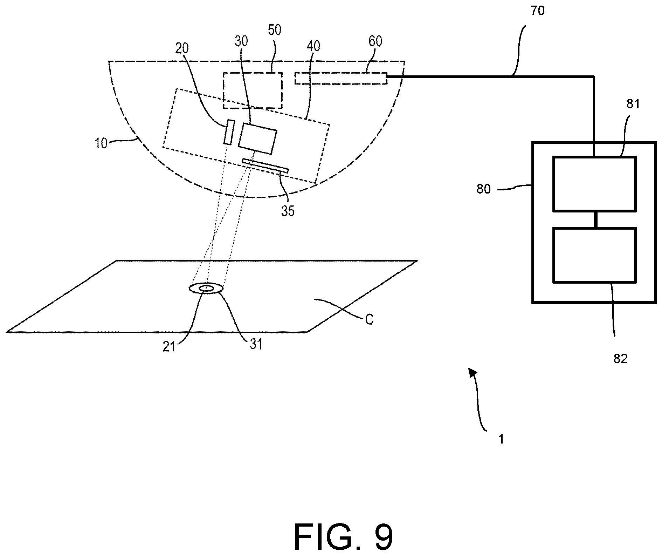

A system suitable for performing the present method is shown in Figures 9 to 12. The system 1 comprises a chlorophyll fluorescence measuring device, which comprises a dome-shaped housing 10, which encloses a laser 20 and a camera or non-imaging optical sensor 30 mounted on a support 40. The support is connected to an actuator 50, which moves the support 40 within the housing 10 so as to change the direction in which the laser 20 and optical sensor 30 are pointed. The housing also encloses an onboard controller 60, which controls the operation of the actuator, laser and optical sensor. The onboard controller 60 is connected by a communication line 70 to a computer system 80 remote from the chlorophyll fluorescence measuring device. The communication line may be a physical communication line or wireless communication means. The computer system 80 comprises a central controller 81, which is connected to the onboard controller 60 by the communication line 70, and also comprises a display screen 82.

As shown in Figure 9, the dome-shaped housing 10 is mounted by its substantially flat base over a crop C of plants being grown in an area below, so that the dome part of the housing projects downwards. In this arrangement, the support 40 is manoeuvred by the actuator 50 to point the laser 20 and optical sensor 30 at a target in the crop C below. The laser 20 irradiates a measurement area 21 with light and is controlled by the controller 60 to emit a saturation or measurement pulse in accordance with the type of measurement being made. The target may be between 0.1 and 1 meter from the system 1, although the system may be capable of working over a much wider range of distances, and the laser spot size at this distance may be around 2 mm in diameter. The optical sensor 30 has a field of view 31 of the canopy which encompasses the measurement area 21 irradiated by the laser light. The optical sensor may have a field of view corresponding to an area of the canopy of around 20 cm$^2$ at the distance of between 0.1 and 1 meter. A filter 35 is positioned between the optical sensor 30 and the target so that the light emitted from the target as chlorophyll fluorescence is detected by the optical sensor 30 and the reflected light from the laser spot 21 is filtered out by the filter 35.

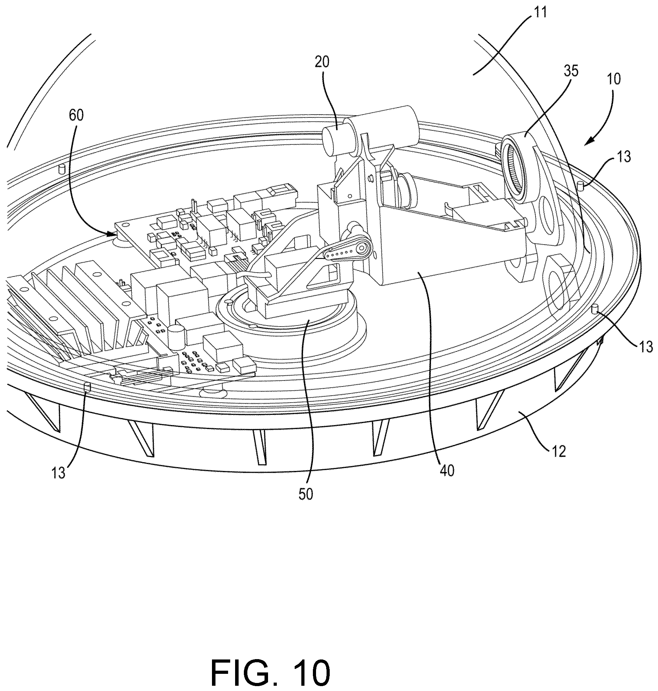

Figure 10 shows the system in more detail with the optical sensor 30 omitted and shows that the dome-shaped housing 10 comprises a transparent dome-shaped cover 11, which is attached to a circular base 12 with a raised rim at its periphery. The cover 11 is attached to the circular base 12 by screws (not shown) that connect through screw holes 13 in a peripheral lip of the cover 11, and fasten the cover 11 to the base 12. The remaining components of the system are then enclosed within the housing, being contained between the base 12 and the cover 11, with the optical components, i.e. the laser 20 and the optical sensor 30 still able to operate through the transparent cover 11. The entire system 1 may be mounted over a canopy by attaching the base 12 to a surface over the canopy, such as the ceiling, via screw holes (not shown) or other attachment means of the base 12.

Within the housing 10 are control electronics 60. This includes a controller for the actuator 50, a controller for the laser 20, and a controller for the optical sensor 30, which are coordinated to take a measurements in the crop C below the system. These control electronics are mounted on the base 12 of the housing 10 so that they are stationary during use.

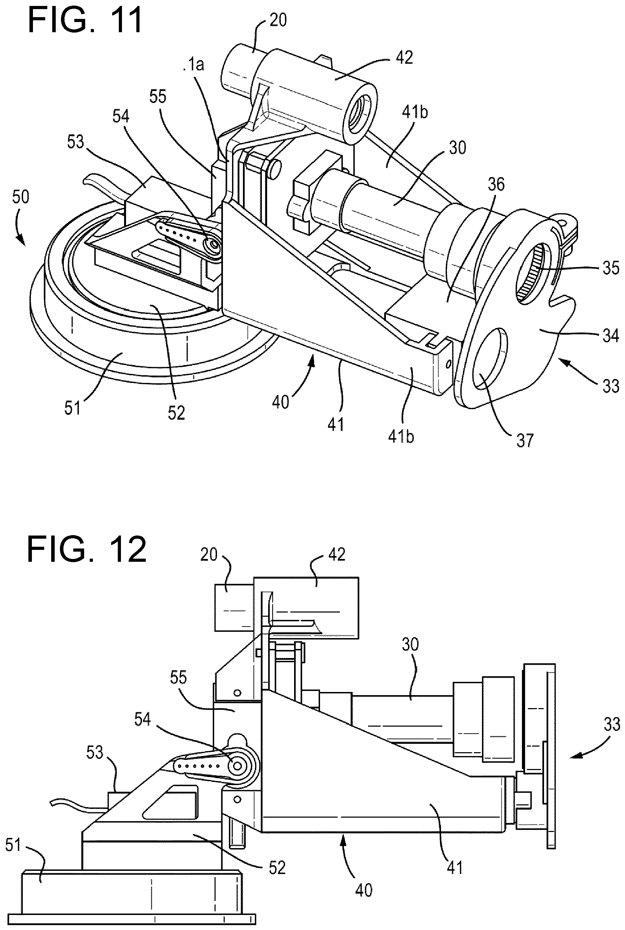

Also within the housing 10, as mentioned, are the laser 20, optical sensor 30, support 40 and actuator 50, which are shown in isolation in Figures 11 and 12. As shown in these figures, in this embodiment the optical sensor is a camera 30. The actuator 50 comprises a ring-shaped base 51 inside of which is mounted a platform 52 having a circular part that is received in the base 51 and a mounting portion. On the mounting portion is located a pan servo motor 53, which operates through a central axis through the platform and is preferably capable of rotating the platform through 360°. Also mounted to the platform 52 via an axis 54 is a tilt servo motor 55. The tilt servo motor is preferably capable of rotating 90° in this configuration, relative to the platform 52. The actuator, in this case, comprises the combination of the pan servo motor 53 and the tilt servo motor 55. An actuator of this type, with variable yaw and pitch, is able to point the laser and the camera across a wide area and so survey a large crop area with a small system footprint.

The support 40 is a piece of moulded plastic that attaches to the housing of the tilt motor 55 of the actuator 50 and supports both of the laser 20 and the camera 30, along with the filter 35. The support 40 comprises, in particular, a camera-holding portion 41 and a laser holding portion 42. These are arranged so that both the laser 20 and the camera 30 are pointed along the same direction, i.e. parallel with one another, with the laser 20 being spaced a few centimetres above the camera 30.

The camera-holding portion 41 includes a rear plate 41a, to which the camera 30 is attached, pointing away from the rear plate and towards the target. It also comprises two arms 41b, which extend forwards from the rear plate 41a, beneath the camera barrel. The two arms connect at their forward end to the filter assembly 33. The filter assembly comprises a filter wheel 34 connected to a filter motor 36 mounted on end of the arms 41b. The filter motor 36 operates to rotate the filter wheel 34 so that either filter 35 or filter 37 within the filter wheel may be placed in front of the camera 30. The filter 35, which is held in front of the lens of the camera, may be a long-pass interference filter, which filters out perpendicularly incident light having a wavelength of less than 650 nm, to allow the camera 30 to measure the fluorescence in the range 650 to 750 nm, or may be a band-pass filter, blocking all light outside of the range 650 to 750 nm. Filter 37 may be a different filter, such as a band-pass filter of 650 to 700 nm or 700 nm to 750 nm, for acquiring PSI or PSII fluorescence in isolation. Alternatively, filter 35 may be a band-pass filter of 650 to 700 nm and filter 37 a band-pass filter of 700 nm to 750 nm to allow the system to measure both PSI and PSII fluorescence independently of one another.

The laser-holding portion 42 of the support 40 is a substantially cylindrical sleeve that is open at both ends, with a smaller opening at the front end, through which the laser beam is emitted. The laser-holding portion 42 receives a substantially cylindrical laser in the open rear end, which points forwards through the open front end.

The laser 20 itself is configured to emit collimated light having a wavelength of approximately 450 nm, with a beam diameter of 3 mm and should be capable of delivering saturation pulses lasting approximately one second to the target having an average photosynthetic photon flux density (PPFD) of 8000 $\mu$mol m$^{-2}$ s$^{-1}$. The laser should also be capable of delivering measurement pulses lasting between 1 $\mu$s and 10 ms.

The camera 30 may be an OV9281 manufactured by OmniVision® of 4275 Burton Drive, Santa Clara, California 95054 USA. This camera may be configured with a relatively small field of view of 6° x 4°, which decreases the noise in the signal and increases the sensitivity to the area irradiated by the laser. As mentioned previously, the field of view of the camera must be large enough for the laser spot to be visible across the working range of the system. In embodiments in which the laser 20 and the camera 30 are pointed parallel to one another, the laser spot will be in the centre of the field of view at infinity, and will be closer to the top of the field of view the closer the target is to the system. The position of the laser in the field of view of the camera, as well as the spot size, can be used to determine the distance to the target. In other systems, the laser and the camera may not be parallel and may instead define a small but fixed angle in the direction they are pointed, so that the laser spot is in the middle of the field of view of the camera roughly in the centre of the working range of the system. This may cause the spot to move across the full field of view of the camera depending on the distance to the target, and so offer greater sensitivity in measuring the distance to the target. An angle of approximately 8° has been found to be suitable for a typical separation distance of camera and laser and for a working distance in the range 0.1 to 1 m, although it will be appreciated that these can be configured as needed depending on the particular system installation, including the working range and the spacing of the camera and laser. In other embodiments, the angle between the laser and the camera could be adjustable. The control system may adjust the angle between the laser 20 and the camera 30 until the spot is in the centre of the field of view, and then the distance to the target determined by the angle between the camera and the laser needed to achieve this centring of the laser spot.

In step S103, it is checked whether measurements have been made with actinic light of ten different intensities. If not, then the method proceeds to step S104 where the set actinic light intensity is temporarily adjusted by adjusting the power of the actinic light source to a new intensity value. This may be done automatically by the system performing the method, if controller 81 is operationally linked to the light source, e.g. by the controller controlling the light source, or a request may be output on a display screen 82 for a grower to adjust the light source. The method then returns to step S102 and another set of measurements is made at a new light intensity value. It should be noted that while this embodiment makes adjustments to the actinic light intensity, this is not essential and the method could alternatively make ten or more different sets of measurements evenly spaced over a period of, for example, 20 to 40 minutes. The plant will naturally fluctuate in efficiency due to factors including water consumption, natural humidity/temperature changes, time of day, etc. such that it is not essential to use the light source to deliberately induce changes, although this can speed up the method and provide more control over the state of the plant being measured.

Once measurements have been made with actinic light of ten different intensities, the method proceeds to step S105. It should be noted here that more or fewer than ten different intensities of light may be used. A greater number of measurements provides more data for the subsequent correlations but takes longer to perform. Also, the present method performs ten different sets of measurements at a single location of the crop, but alternatively, the method could repeat the set of measurements at a number of measurement locations of the crop for each light intensity setting.

In step S105, for each set of measurements, the quantum yield of photosynthesis of the plant is estimated. This may be done by calculating $\dfrac{F_m{'}-F{'}}{F_m{'}}$. If measurements were taken at a number of different locations for each light intensity value, then these should be averaged for each light intensity setting. The result should be an estimated quantum yield for each of the ten different actinic light intensities.

In step S106, a maximum efficiency of PSII photochemistry is estimated for each set of measurements. This may be done by calculating $\dfrac{F_m{'}-F_0{'}}{F_m{'}}$. In step S107, a photochemical quenching coefficient is estimated. This may be done by calculating $\dfrac{F_m{'}-F{'}}{F_m{'}-F_0{'}}$. Again, if measurements were taken at a number of different locations for each light intensity value, then these should be averaged for each light intensity setting. The result will be an estimated maximum efficiency of PSII photochemistry and an estimated photochemical quenching coefficient for each of the ten different actinic light intensities.

In step S108, the data is correlated. This step first comprises taking logarithms of each estimated value, i.e. $\log\frac{F_m{}'-F'}{F_m{}'}$, $\log\frac{F_m{}'-F_0{}'}{F_m{}'}$, and $\log\frac{F_m{}'-F'}{F_m{}'-F_0{}'}$. Then a first Pearson correlation is performed correlating the $\log\frac{F_m{}'-F'}{F_m{}'}$ values with the $\log\frac{F_m{}'-F_0{}'}{F_m{}'}$ values. Finally, a second Pearson correlation is performed correlating the $\log\frac{F_m{}'-F'}{F_m{}'}$ values with the $\log\frac{F_m{}'-F'}{F_m{}'-F_0{}'}$ values.

The estimations and the correlations of steps S105 to S108 may be performed using the central controller 81, which receives the measurement data from the controller 60 via the communication line 70.

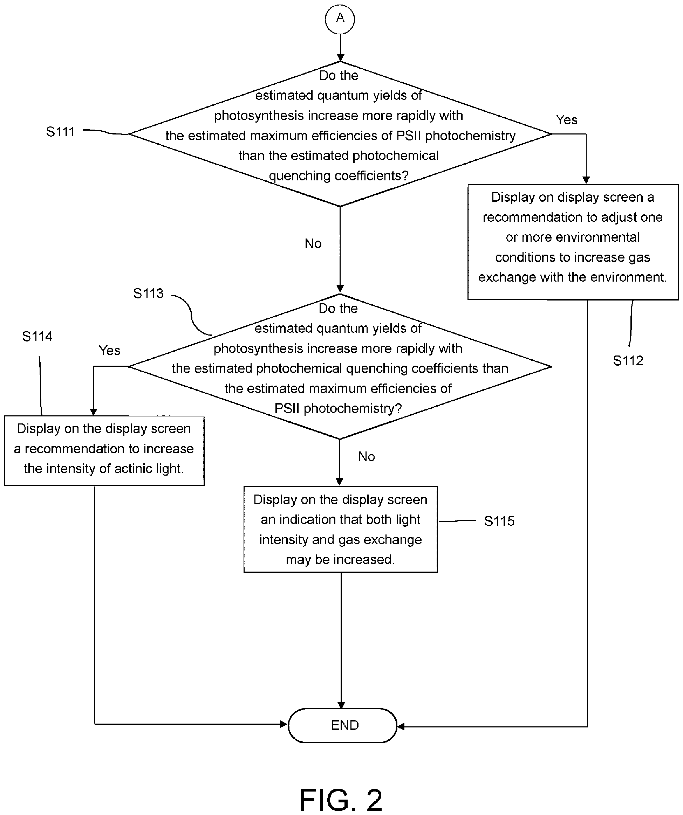

The correlations calculated in step S108 allow for it to be determined whether a plant is light limited or $CO_2$ limited. In some embodiments of the method, the steps of Figure 1 could be followed by a step of displaying on the display screen 82 the results of the two correlations. For example, the correlations may be graphically presented on the display screen 82, so that a grower can make a decision as to how to adjust the environmental conditions in line with their objectives. However, preferably the method further comprises performing a comparison of the data and making or recommending adjustments based on that comparison. There are several different ways that this comparison can be performed and ways that the results can be acted upon by the system, some examples of which will be described with reference to Figures 2 to 8, which illustrate several variations of a second part of the method that may follow on from the first part illustrated in Figure 1.

The flow diagram of Figure 2 begins with step S111, which follows on from step S108 of Figure 1. In this step, it is determined whether the estimated quantum yields of photosynthesis increase more rapidly with the estimated maximum efficiencies of PSII photochemistry than the estimated photochemical quenching coefficients. This can be determined by looking at the slopes of the Pearson correlations obtained in step S108. If the estimated quantum yields of photosynthesis does increase more rapidly with the estimated maximum efficiencies of PSII photochemistry than the estimated photochemical quenching coefficients, then it is established that the plant is $CO_2$ limited and the method proceeds to step S112. In step S112, the controller 81 outputs a control signal, which in this case causes a display screen 82 of the system to display a recommendation to adjust one or more environmental conditions to increase gas exchange with the environment. The process then ends.

If, however, at step S111 it is determined that the estimated quantum yields of photosynthesis does not increase more rapidly with the estimated maximum efficiencies of PSII photochemistry than the estimated photochemical quenching coefficients, then the method moves on to step S113, in which it is checked whether the estimated quantum yields of photosynthesis increase more rapidly with the estimated photochemical quenching coefficients than the estimated maximum efficiencies of PSII photochemistry. Again, this is determined by looking at the slopes of the Pearson correlations obtained in step S108. If the estimated quantum yields of photosynthesis does increase more rapidly with the estimated photochemical quenching coefficients than the estimated maximum efficiencies of PSII photochemistry, then it is established that the plant is light limited, and the method proceeds to step S114, in which the controller 81 outputs a control signal, which in this case causes the display screen 82 to display a recommendation to increase the intensity of actinic light. The process then ends.

In the rare case that it is determined in step S113 that the estimated quantum yields of photosynthesis does not increase more rapidly with the estimated photochemical quenching coefficients than the estimated maximum efficiencies of PSII photochemistry, then the calculated slopes of the correlations must be the same, and the crop is well balanced. Accordingly, the method may proceed to step S115, in which the controller 81 outputs a control signal to cause the display screen 82 to indicate that both light intensity and gas exchange should be increased. The process then ends.

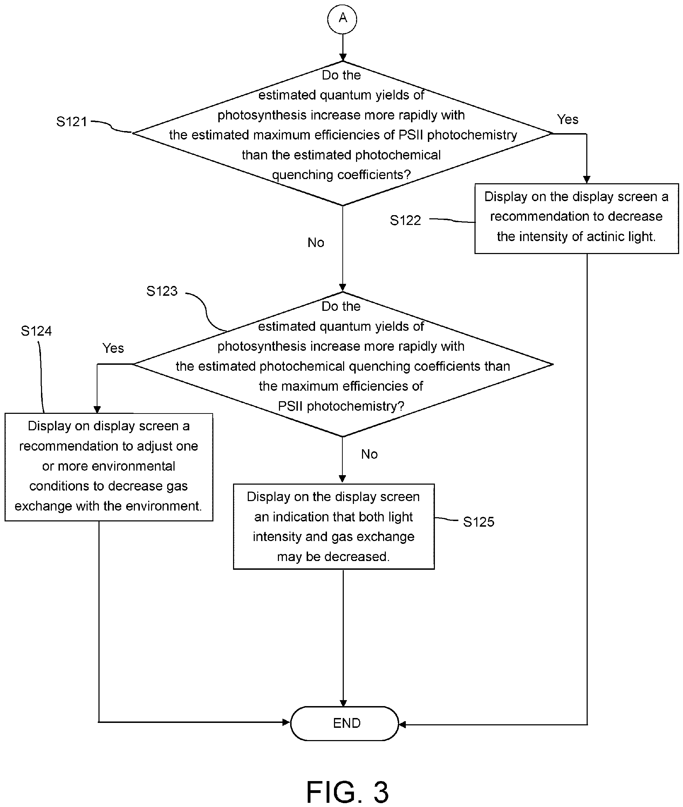

The above method assumes that the grower is intending to increase plant growth. This preference may be input via the display screen 82, which may be used to initiate the method. However, it is also possible that the grower may want to decrease resource usage. Figure 3 shows a variant of the method of Figure 2, in which in a step S121, which follows on from step S108 of Figure 1, it is determined whether the estimated quantum yields of photosynthesis increase more rapidly with the estimated maximum efficiencies of PSII photochemistry than the estimated photochemical quenching coefficients, and if it does, it proceeds to step S122, in which the controller 81 outputs a control signal to cause the display screen 82 to display a recommendation to decrease the intensity of actinic light.

If, in step S121, it is determined that the estimated quantum yields of photosynthesis does not increase more rapidly with the estimated maximum efficiencies of PSII photochemistry than the estimated photochemical quenching coefficients, then the method moves to step S123, in which it is checked whether the estimated quantum yields of photosynthesis increase more rapidly with the estimated photochemical quenching coefficients than the estimated maximum efficiencies of PSII photochemistry. If they do, then the method moves to step S124, in which the controller 81 causes the display screen 82 to output a recommendation to adjust one or more environmental conditions to decrease gas exchange with the environment.

Again, in the rare case that step S123 also does not find that the estimated quantum yields of photosynthesis increase more rapidly with the estimated photochemical quenching coefficients than the estimated maximum efficiencies of PSII photochemistry, then light and $CO_2$ are well balanced and the controller 81 causes the display screen 82 to indicate that both light intensity and gas exchange may be decreased.

Of course, the above methods could be combined, so that if the crop is found to be $CO_2$ limited, then the display screen recommends either adjusting one or more environmental conditions to increase gas exchange with the environment or decreasing light intensity, or if the crop is found to be light limited, then the display screen recommends either increasing light intensity or adjusting one or more environmental conditions to decrease gas exchange with the environment.

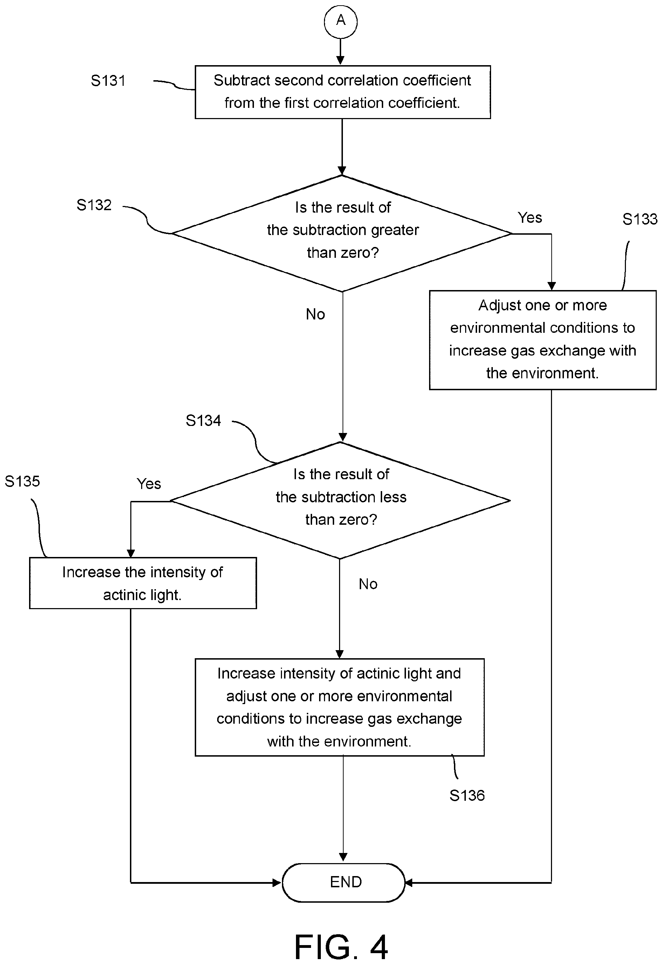

Figure 4 shows another alternative second part to the method, which follows on from the part shown in Figure 1. This differs from Figure 2 in two respects: firstly, in how the correlations are compared and secondly in how the result of the comparison is acted upon, as will be described below.

Firstly, in Figure 4, in step S131, the correlation coefficient of the second correlation is subtracted from the correlation coefficient of the first correlation. Then, in step S132, it is checked whether the result of the subtraction is greater than zero. It can be assumed that if the crop is $CO_2$ limited, then there will be a stronger correlation between quantum yield and the maximum efficiencies of PSII photochemistry than between quantum yield and the photochemical quenching coefficients. Therefore, the result of this subtraction being greater than zero is a strong indicator that the crop is $CO_2$ limited. Thus, if the value is greater than zero, the method proceeds to step S133.

In step S133, one or more environmental conditions are automatically adjusted by the controller 81 in order to increase gas exchange with the environment. This method requires that the controller 81 is operationally linked with one or more systems for controlling conditions such as temperature, humidity, pressure and $CO_2$ concentration. Unlike the previous methods, this removes the need to display on a display screen a recommendation to a grower for them to make manual adjustments. This method may require, however, that the grower input, e.g. via the display screen 82, an indication as to whether crop growth rate should be increased or decreased, with the method shown assuming that rate is to be increased.

If the result of the subtraction is not greater than zero, then in step S134 it is checked whether the result of the subtraction is less than zero. If it is, then in step S135, the controller automatically increases the intensity of the actinic light. Again, this requires that the controller is operationally linked with an adjustable light source and also that the grower has input an indication that crop growth rate should be increased.

In the rare case that the result of the subtraction is precisely zero, from S134, the method proceeds to step S136, in which the system increases both light intensity and one or more environmental conditions to increase gas exchange with the environment. Alternatively, the method could proceed to look at the slope of the correlations to identify which correlation has the greater slope.

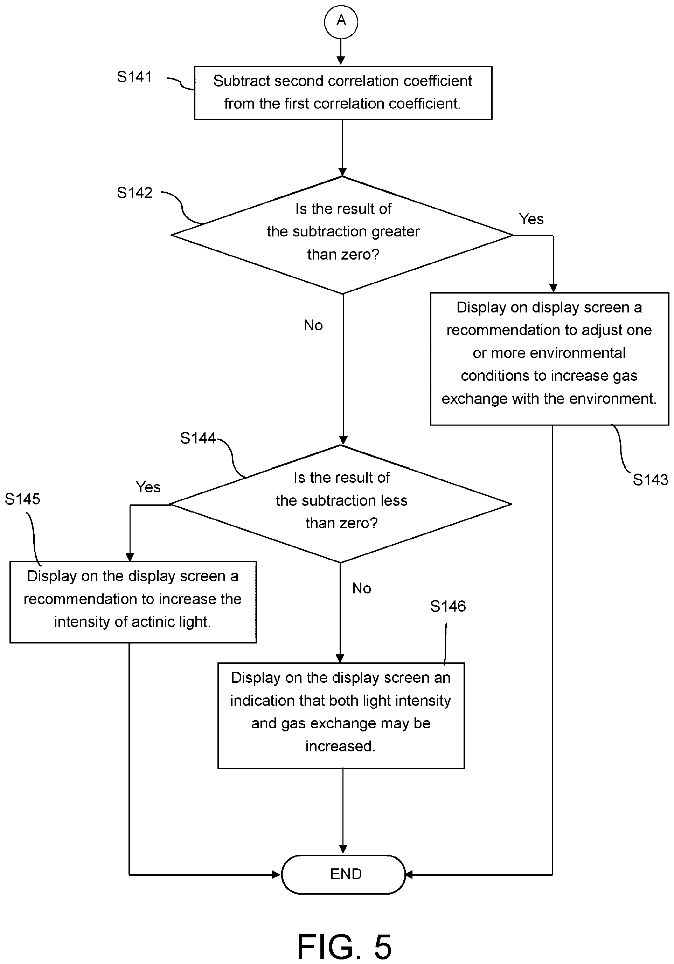

The method illustrated by Figure 5 is similar to Figure 4, in that it begins with a step S141, which the correlation coefficient of the second correlation is subtracted from the correlation coefficient of the first correlation. Then, in step S142, it is checked whether the result of the subtraction is greater than zero. If the result is greater than zero, then in step S143, the controller 81 causes a display screen 82 to display a recommendation to adjust one or more environmental conditions to increase gas exchange with the environment. Likewise, if, in step S144, it is determined that the result of the subtraction is less than zero, then in step S145, the controller 81 causes a display screen 82 to display a recommendation to increase the intensity of actinic light. If the result of the subtraction is precisely zero, then in step S146, the controller 81 causes the display screen 82 to display an indication that both light intensity and gas exchange may be increased.

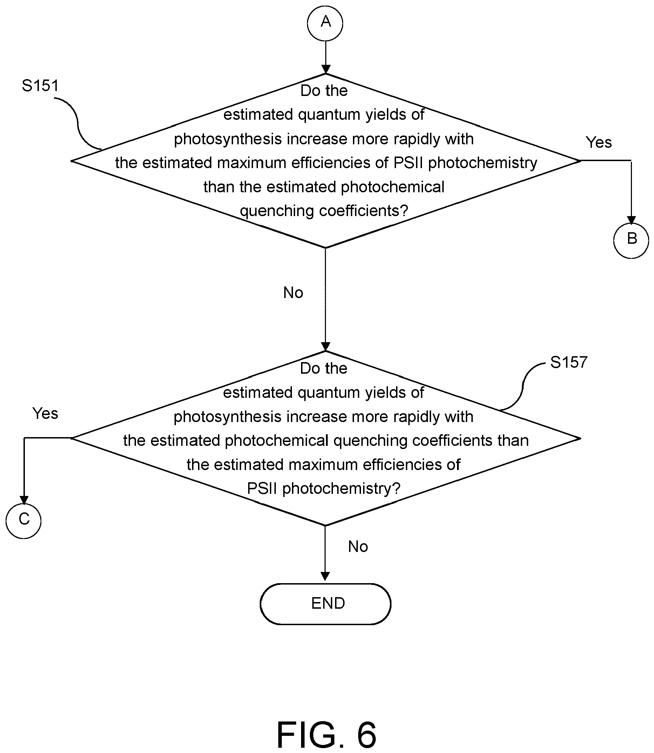

The above methods assume no knowledge of current light intensity values or of the conditions such as temperature, humidity, pressure and $CO_2$ concentration. However, it will be appreciated that these may also be measured and factored into the control of the environmental conditions in a number of ways. Figures 6 to 8 illustrate one such method, in which the part of the method illustrated in these Figures again follows on from Figure 1. In this method, the system is set up to automatically adjust environmental conditions in order to maintain a good balance between light intensity and gas exchange between the plant and the environment. This method requires that a grower input a minimum and maximum light intensity, which may vary depending on the grower's objectives.

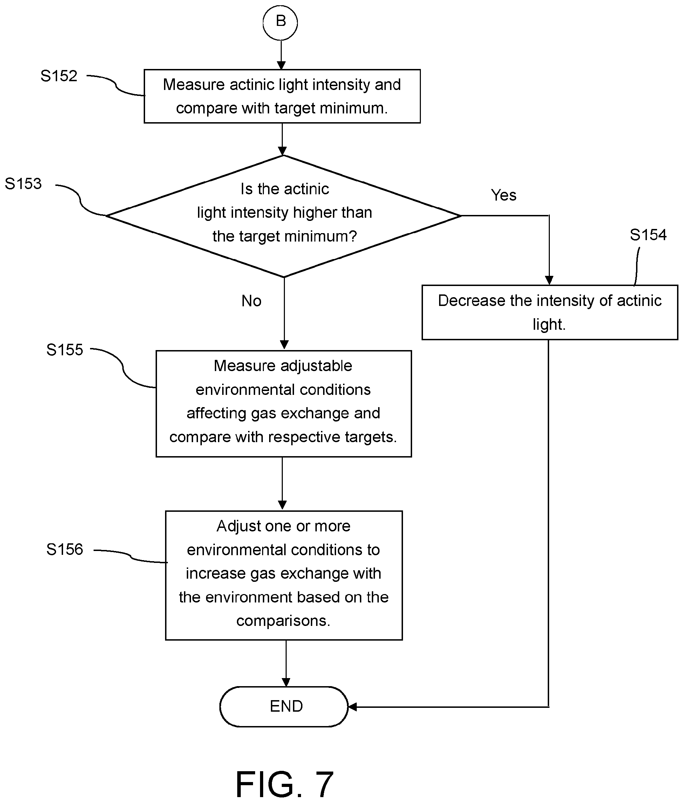

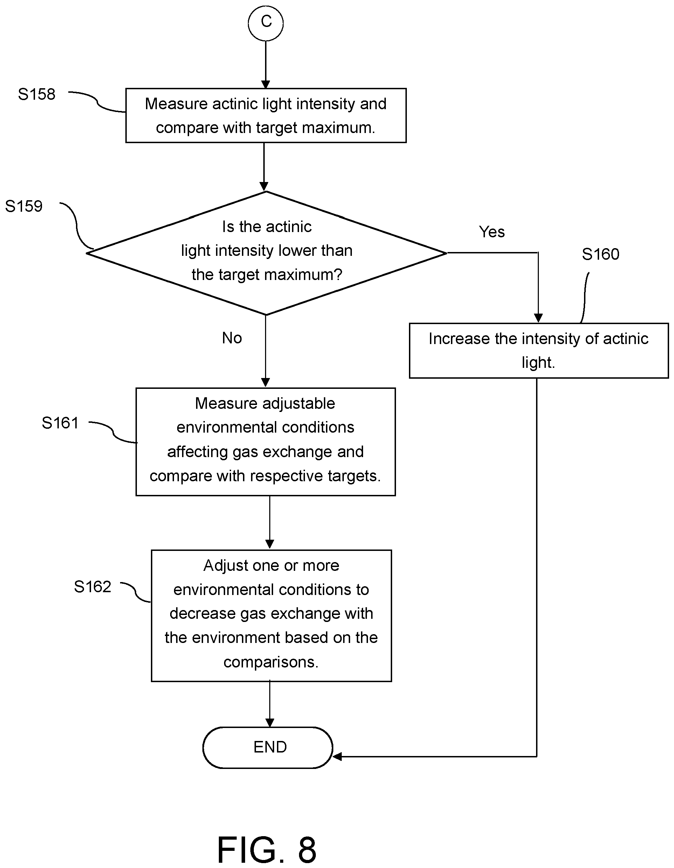

Figure 6 begins with step S151, which follows on from step S108 in Figure 8. In this step, it is determined whether the estimated quantum yields of photosynthesis increase more rapidly with the estimated maximum efficiencies of PSII photochemistry than the estimated photochemical quenching coefficients. Again, this may be done by assessing the slope of the correlations. If the estimated quantum yields of photosynthesis does increase more rapidly with the estimated maximum efficiencies of PSII photochemistry than the estimated photochemical quenching coefficients, i.e. the crop is $CO_2$ limited, then the method proceeds to step S152, shown in Figure 7.

In step S152, actinic light currently being used to grow the crop (i.e. not one of the temporary changes made in step S104) is measured and compared with the target minimum light intensity. The actinic light intensity may be measured with a PPFD sensor. Alternatively, light intensity may be determined using the camera of the chlorophyll fluorescence measurement device. In order to determine the light intensity from an image captured by the camera, the present method models changes in light intensity as a linear relationship with changes in light reflection from plant leaves on grayscale camera images. Computer vision may be used to identify an area of the image containing a leaf (and not containing the laser spot if the image is a measurement image). The greyscale pixel brightnesses may be averaged across this leaf area in order to get an average pixel brightness, which will be used as an indicator of the actinic light intensity. It should be noted that if pulse width modulated grow lights are used, then the image used to determine light intensity should have been captured with an exposure time long enough to view at least one full cycle of the grow lights, preferably a plurality of cycles, so that the average pixel value truly reflects the intensity of irradiating light. This method may require that calibration images are taken in order to establish average pixel values with the actinic light source at 0% and 100% intensity. As an alternative to measuring light intensity, if the controller 81 is operationally connected to the adjustable light source, it may simply obtain the current light control setting.

In step S153, it is determined whether the actinic light intensity higher than the target minimum. If it is, the method proceeds to step S154, in which the intensity of actinic light is decreased. Instead of decreasing the light intensity, the method could output a recommendation to do so.

If the actinic light is already at the target minimum, then balance cannot be achieved by adjusting the light source, and so instead the method proceeds to step S155. In this step, the system measures environmental conditions affecting gas exchange. This will typically comprise measuring any adjustable conditions using a sensor, for example a temperature sensor, humidity sensor, pressure sensor or $CO_2$ concentration sensor. Then, in step S156, the method involves adjusting one or more of these conditions to increase gas exchange with the environment based on the comparisons. For example, this may comprise increasing the parameter that is currently lowest compared with a preset working range for those parameters. Alternatively, the method could involve displaying a recommendation to adjust one of the environmental conditions based on the measurements.

Returning to Figure 6, if the quantum yields of photosynthesis do not increase more rapidly with the estimated maximum efficiencies of PSII photochemistry than the estimated photochemical quenching coefficients, then the method proceeds to step S157, in which the reverse is checked. If the estimated quantum yields of photosynthesis increase more rapidly with the estimated photochemical quenching coefficients than the estimated maximum efficiencies of PSII photochemistry, then the plant is light limited and the method proceeds to step S158, shown in Figure 8. On the other hand, if this criterion is not fulfilled, then light and $CO_2$ are well ba-

lanced, and no intervention is required.

Step S158, shown in Figure 8, comprises measuring actinic light intensity and comparing with a target maximum. The measurement may be made in the same manner as S152. If the light intensity is lower than the target maximum, then the method proceeds to step S160, in which the intensity of light is increased. Alternatively, a recommendation could be displayed on a display to increase light intensity.

If the light intensity is already at the target maximum, then the balance cannot be achieved by adjusting the light intensity, and so the method proceeds to step S161, in which he system measures environmental conditions affecting gas exchange, analogously to step S155. In following step S162, the system adjusts one or more environmental conditions to decrease gas exchange with the environment based on the comparisons. Again, this could comprise decreasing the parameter that is currently highest compared with a preset working range for those parameters. Alternatively, this step could involve displaying a recommendation to adjust one of the environmental conditions based on the measurements.

To prevent the method from making adjustments back and forth once the light and $CO_2$ are reasonably well balanced, steps S151 and S157 could check whether the difference in the rate of increase of the quantum yields with the estimated maximum efficiencies of PSII photochemistry and the estimated photochemical quenching coefficients is greater than a threshold difference. Alternatively, the method could check which the quantum yields increased more rapidly with in a previous iteration of the method and if the opposite is now found, no adjustment made until a preset time has elapsed.

**Claims**

1. A method of controlling environmental conditions for plant growth, the method comprising:

   making a plurality of sets of chlorophyll fluorescence measurements of a plant at plurality of different measurement times;
   calculating a first parameter indicative of a quantum yield of photosynthesis of the plant at each measurement time based on the respective set of chlorophyll fluorescence measurements;
   calculating a second parameter indicative of a maximum efficiency of PSII photochemistry of the plant at a plurality of the measurement times based on the respective set of chlorophyll fluorescence measurements;
   calculating a third parameter indicative of a photochemical quenching coefficient of the plant at a plurality of the measurement times based on the respective set of chlorophyll fluorescence measurements; and,
   in a comparison step,

ascertaining a first relationship between the first and second parameters indicative of how the quantum yields of photosynthesis vary with the maximum efficiencies of PSII photochemistry; ascertaining a second relationship between the first and third parameters indicative of how the quantum yields of photosynthesis vary with the photochemical quenching coefficients; and either:

generating a control signal indicative of plant growth being limited by gas exchange with the environment when the first and second relationships indicate that the quantum yields of photosynthesis increase more rapidly with, or are more strongly positively correlated to, the maximum efficiencies of PSII photochemistry than the photochemical quenching coefficients; or generating a control signal indicative of plant growth being limited by light exposure from the environment when the first and second relationships indicate that the quantum yields of photosynthesis increase more rapidly with, or are more strongly positively correlated to, the photochemical quenching coefficients than the maximum efficiencies of PSII photochemistry.

2. A method according to claim 1, wherein ascertaining the first relationship comprises performing a first correlation, which comprises correlating the first parameter with the second parameter, and wherein ascertaining the second relationship comprises performing a second correlation, which comprises correlating the first parameter with the third parameter, and wherein the comparison step comprises comparing the first and second correlations, wherein preferably the first and second correlations are Pearson correlations, wherein preferably the comparison step comprises comparing a correlation coefficient from the first correlation with a correlation coefficient from the second correlation, and comprises either: generating the control signal indicative of plant growth being limited by gas exchange with the environment when the first correlation coefficient is greater than the second correlation coefficient; or generating the control signal indicative of plant growth being limited by light exposure from the environment when the second correlation coefficient is greater than the first correlation coefficient.

3. A method according to any of the preceding claims, further comprising irradiating a plant with actinic light using an adjustable light source before and/or while making at least some of the plurality of the sets of chlorophyll fluorescence measurements, wherein the intensity of actinic light for at least two of those sets of chlorophyll fluorescence measurements is different, wherein preferably the intensity of actinic light for each of those sets of chlorophyll fluorescence measurements is different.

4. A method according to any of the preceding claims, wherein each set of chlorophyll fluorescence measurements is at least 30 seconds apart, preferably at least one minute apart.

5. A method according to any of the preceding claims, further comprising irradiating a plant with actinic light having a set intensity using an adjustable light source before making the plurality of sets of chlorophyll fluorescence measurements, and wherein generating a control signal indicative of plant growth being limited by gas exchange with the environment comprises generating a control signal to decrease the set intensity of actinic light, and/or wherein generating a control signal indicative of plant growth being limited by light exposure from the environment comprises generating a control signal to increase the set intensity of actinic light.

6. A method according to any of the preceding claims, wherein generating a control signal indicative of plant growth being limited by gas exchange with the environment comprises generating a control signal to adjust one or more environmental conditions to increase gas exchange with the environment, and/or wherein generating a control signal indicative of plant growth being limited by light exposure from the environment comprises generating a control signal to adjust one or more environmental conditions to decrease gas exchange with the environment.

7. A method according to any of the preceding claims, wherein generating a control signal indicative of plant growth being limited by gas exchange with the environment comprises generating a control signal that causes output of a recommendation to decrease an actinic light intensity and/or to adjust one or more environmental conditions to increase gas exchange with the environment, and/or wherein generating a control signal indicative of plant growth being limited by light exposure from the environment comprises generating a control signal that causes output of a recommendation to increase an actinic light intensity and/or to adjust one or more environmental conditions so as to decrease gas exchange with the environment.

8. A method according to claim 6 or claim 7, wherein the one or more environmental conditions include one or more of temperature, humidity, pressure, and $CO_2$ concentration of the environment.

9. A method according to claim 8, wherein the method further comprises taking one or more measurements of the environment, said one or more measurements including one or more of taking a temperature measurement of the environment using a temperature sensor, taking a humidity measurement of the environment using a humidity sensor, taking a pressure measurement of the environment using a pressure sensor, and taking a $CO_2$ concentration measurement of the environment using a $CO_2$ concentration sensor, and wherein generating a control signal to adjust one or more environmental conditions or generating a control signal that causes output of a recommendation to adjust one or more environmental conditions is further based on the one or more measurements of the environment.

10. A method according to claim 5 or claim 7, or any claim dependent thereon, further comprising taking a measurement of the intensity of actinic light, and wherein generating a control signal to increase or decrease the set intensity of actinic light or generating a control signal that causes output of a recommendation to increase or decrease an actinic light intensity is further based on the measurement of the intensity of actinic light.

11. A method according to any of the preceding claims, wherein generating a control signal indicative of plant growth being limited by gas exchange with the environment comprises generating a control signal that causes output of a visual and/or audible indication that plant growth is limited by gas exchange with the environment, and/or wherein generating a control signal indicative of plant growth being limited by light exposure from the environment comprises generating a control signal that causes output of a visual and/or audible indication that plant growth is limited by light exposure from the environment, wherein preferably the or each visual indication comprises displaying information on a display screen.

12. A method according to any of the preceding claims, wherein at least two of the sets of chlorophyll fluorescence measurements include a measurement of a maximal fluorescence of the plant in a light-adapted state, $F_m'$, and a baseline fluorescence of the plant in a light-adapted state, F', and preferably a minimal fluorescence of the plant in a light-adapted state, $F_0'$, wherein preferably each set of chlorophyll fluorescence measurements includes a measurement of the maximal fluorescence of the plant in a light-adapted state, $F_m'$, and the baseline fluorescence of the plant in a light-adapted state, F', and preferably a measurement of a minimal fluorescence of the plant in a light-adapted state, $F_0'$.

13. A method according to claim 12, comprising either making a measurement of the minimal fluorescence of the plant in a light-adapted state, $F_0'$, in at least two of the sets of chlorophyll fluorescence measurements, or estimating the minimal fluorescence of the plant in a light-adapted state, $F_0'$, based on other chlorophyll fluorescence measurements of at least two of the sets of chlorophyll fluorescence measurements, and wherein calculating the first parameter comprises estimating the quantum yield of photosynthesis for each measurement time, by calculating $\dfrac{F_m'-F'}{F_m'}$, and/or wherein calculating the second parameter comprises estimating the maximum efficiency of PSII photochemistry for each measurement time, by calculating $\dfrac{F_m'-F_0'}{F_m'}$, and/or wherein calculating the third parameter comprises estimating the photochemical quenching coefficient for each measurement time, by calculating $\dfrac{F_m'-F'}{F_m'-F_0'}$.

14. A system for controlling environmental conditions for plant growth, the system comprising:

   a chlorophyll fluorescence measurement device configured to make a plurality of sets of chlorophyll fluorescence measurements of a plant at plurality of different measurement times; and
   a controller, the controller configured to:

      calculate a first parameter indicative of a quantum yield of photosynthesis of the plant at each measurement time based on the respective set of chlorophyll fluorescence measurements;
      calculate a second parameter indicative of a maximum efficiency of PSII photochemistry of the plant at a plurality of the measurement times based on the respective set of chlorophyll fluorescence measurements;
      calculate a third parameter indicative of a photochemical quenching coefficient of the plant at a plurality of the measurement times based on the respective set of chlorophyll fluorescence measurements; and
      to perform a comparison comprising ascertaining a first relationship between the first and second parameters indicative of how the quantum yields of photosynthesis vary with the maximum efficiencies of PSII photochemistry, ascertaining a second relationship between the first and third parameters indicative of how the quantum yields of photosynthesis vary with the photochemical quenching coefficients, and either: generate a control signal indicative of plant growth being limited by gas exchange with the environment when the first and second relationships indicate that the quantum yields of photosynthesis increase more rapidly with, or are more strongly positively correlated to, the maximum efficiency of PSII photochemistry than the photochemical quenching coefficients; or generate a control signal indicative of plant growth being limited by light exposure from the environment when the first and second relationships indicate that the quantum yields of photosynthesis increase more rapidly with, or are more strongly positively correlated to, the photochemical quenching coefficients than the maximum efficiency of PSII photochemistry.

15. A method of controlling environmental conditions for plant growth, the method comprising:

making a plurality of sets of chlorophyll fluorescence measurements of a plant at plurality of different measurement times;
calculating a first parameter indicative of a quantum yield of photosynthesis of the plant at each measurement time based on the respective set of chlorophyll fluorescence measurements;
calculating a second parameter indicative of a maximum efficiency of PSII photochemistry of the plant at a plurality of the measurement times based on the respective set of chlorophyll fluorescence measurements;
calculating a third parameter indicative of a photochemical quenching coefficient of the plant at a plurality of the measurement times based on the respective set of chlorophyll fluorescence measurements;
ascertaining a first relationship between the first and second parameters indicative of how the quantum yields of photosynthesis vary with the maximum efficiencies of PSII photochemistry;
ascertaining a second relationship between the first and third parameters indicative of how the quantum yields of photosynthesis vary with the photochemical quenching coefficients; and
displaying on a display screen the ascertained first relationship and displaying on the display screen the ascertained second relationship.

START

S101 — Grow a plant using initial set of environmental conditions.

S102 — Make chlorophyll fluorescence measurements $F_0'$, $F_m'$ and $F'$.

S103 — Have measurements been made with actinic light of ten different intensities?

No → Adjust actinic light intensity.

S104

Yes

S105 — Estimate a quantum yield of photosynthesis of the plant for each set of chlorophyll fluorescence measurements.

S106 — Estimate a maximum efficiency of PSII photochemistry of the light-adapted plant for each set of chlorophyll fluorescence measurements.

S107 — Estimate a photochemical quenching coefficient of the plant for each set of chlorophyll fluorescence measurements.

S108 — Perform a first correlation of the estimated quantum yields of photosynthesis with the estimated maximum efficiencies of PSII photochemistry and perform a second correlation of the estimated quantum yields of photosynthesis with the estimated photochemical quenching coefficients.

A

# FIG. 1

S111 — Do the estimated quantum yields of photosynthesis increase more rapidly with the estimated maximum efficiencies of PSII photochemistry than the estimated photochemical quenching coefficients?

Yes →

Display on display screen a recommendation to adjust one or more environmental conditions to increase gas exchange with the environment.

S112

No ↓

S113 — Do the estimated quantum yields of photosynthesis increase more rapidly with the estimated photochemical quenching coefficients than the estimated maximum efficiencies of PSII photochemistry?

Yes →

S114

Display on the display screen a recommendation to increase the intensity of actinic light.

No ↓

Display on the display screen an indication that both light intensity and gas exchange may be increased. — S115

END

FIG. 2

FIG. 3

(A)

S131 — Subtract second correlation coefficient from the first correlation coefficient.

S132 — Is the result of the subtraction greater than zero?

Yes

S133 — Adjust one or more environmental conditions to increase gas exchange with the environment.

No

S134 — Is the result of the subtraction less than zero?

S135 — Increase the intensity of actinic light.

Yes

No

Increase intensity of actinic light and adjust one or more environmental conditions to increase gas exchange with the environment.

S136

END

FIG. 4

FIG. 5

FIG. 6

B

S152 — Measure actinic light intensity and compare with target minimum.

S153 — Is the actinic light intensity higher than the target minimum?

Yes

S154 — Decrease the intensity of actinic light.

No

S155 — Measure adjustable environmental conditions affecting gas exchange and compare with respective targets.

S156 — Adjust one or more environmental conditions to increase gas exchange with the environment based on the comparisons.

END

FIG. 7

S158 — **Measure actinic light intensity and compare with target maximum.**

S159 — **Is the actinic light intensity lower than the target maximum?**

Yes

S160 — **Increase the intensity of actinic light.**

No

S161 — **Measure adjustable environmental conditions affecting gas exchange and compare with respective targets.**

S162 — **Adjust one or more environmental conditions to decrease gas exchange with the environment based on the comparisons.**

END

# FIG. 8

FIG. 9

FIG. 10

# FIG. 11

# FIG. 12

Europäisches
Patentamt

European
Patent Office

Office européen
des brevets

# EUROPEAN SEARCH REPORT

Application Number

EP 23 19 7453

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | WO 2008/118080 A1 (HELIOSPECTRA AKTIEBOLAG [SE]; DUBE SYLVAIN [SE]) 2 October 2008 (2008-10-02) * claim 1; figure 1 * * page 16, line 17 - page 19, line 16 * * page 14, lines 3-26 * | 1-15 | INV. A01G7/04 G01N21/64 G01N33/00 G01N21/84 |
| A | WO 00/25114 A1 (DEUTSCH ZENTR LUFT & RAUMFAHRT [DE]; LUEDEKER WILHELM [DE] ET AL.) 4 May 2000 (2000-05-04) * figure 4 * * page 19, line 14 - page 21, line 2 * | 1,14,15 | |
| A | WO 2004/040274 A1 (PLANT RES INT BV [NL]; JALINK HENDRIK [NL] ET AL.) 13 May 2004 (2004-05-13) * claim 1; figure 1 * | 14 | |
| A | ATHERTON J ET AL: "Using spectral chlorophyll fluorescence and the photochemical reflectance index to predict physiological dynamics", REMOTE SENSING OF ENVIRONMENT, ELSEVIER, XX, vol. 176, 22 January 2016 (2016-01-22), pages 17-30, XP029440998, ISSN: 0034-4257, DOI: 10.1016/J.RSE.2015.12.036 * page 19 - page 20 * | 1,12,14,15 | TECHNICAL FIELDS SEARCHED (IPC) A01G G01N |

The present search report has been drawn up for all claims

| Place of search | Date of completion of the search | Examiner |
|---|---|---|
| The Hague | 5 March 2024 | Guillem Gisbert, I |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 23 19 7453

This annex lists the patent family members relating to the patent documents cited in the above-mentioned European search report.
The members are as contained in the European Patent Office EDP file on
The European Patent Office is in no way liable for these particulars which are merely given for the purpose of information.

05-03-2024

| Patent document cited in search report | | | Publication date | Patent family member(s) | | | Publication date |
|---|---|---|---|---|---|---|---|
| WO 2008118080 | A1 | 02-10-2008 | | CA | 2679330 | A1 | 02-10-2008 |
| | | | | CN | 101636076 | A | 27-01-2010 |
| | | | | DK | 2129212 | T3 | 29-03-2016 |
| | | | | EP | 2129212 | A1 | 09-12-2009 |
| | | | | HK | 1141944 | A1 | 17-12-2010 |
| | | | | JP | 5740762 | B2 | 01-07-2015 |
| | | | | JP | 2010521964 | A | 01-07-2010 |
| | | | | KR | 20100014558 | A | 10-02-2010 |
| | | | | KR | 20150016638 | A | 12-02-2015 |
| | | | | RU | 2009133037 | A | 27-04-2011 |
| | | | | US | 2010115830 | A1 | 13-05-2010 |
| | | | | WO | 2008118080 | A1 | 02-10-2008 |
| WO 0025114 | A1 | 04-05-2000 | | AT | E218705 | T1 | 15-06-2002 |
| | | | | AU | 752897 | B2 | 03-10-2002 |
| | | | | CA | 2348594 | A1 | 04-05-2000 |
| | | | | DE | 69805862 | T2 | 16-01-2003 |
| | | | | EP | 1125111 | A1 | 22-08-2001 |
| | | | | ES | 2174521 | T3 | 01-11-2002 |
| | | | | UA | 66892 | C2 | 15-06-2004 |
| | | | | US | 6563122 | B1 | 13-05-2003 |
| | | | | WO | 0025114 | A1 | 04-05-2000 |
| WO 2004040274 | A1 | 13-05-2004 | | AT | E332497 | T1 | 15-07-2006 |
| | | | | AU | 2003277742 | A1 | 25-05-2004 |
| | | | | BR | 0315850 | A | 20-09-2005 |
| | | | | CA | 2503711 | A1 | 13-05-2004 |
| | | | | DE | 60306675 | T2 | 12-07-2007 |
| | | | | DK | 1563282 | T3 | 30-10-2006 |
| | | | | EP | 1563282 | A1 | 17-08-2005 |
| | | | | ES | 2268487 | T3 | 16-03-2007 |
| | | | | JP | 2006504956 | A | 09-02-2006 |
| | | | | NL | 1021800 | C2 | 06-05-2004 |
| | | | | NZ | 539574 | A | 27-10-2006 |
| | | | | US | 2006102851 | A1 | 18-05-2006 |
| | | | | WO | 2004040274 | A1 | 13-05-2004 |

EPO FORM P0459

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **E. MURCHIE** ; **T. LAWSON**. Chlorophyll fluorescence analysis: a guide to good practice and understanding some new applications.. *Journal of experimental botany*, 2013, vol. 64 (13), 3983-98